# EUROPEAN PATENT APPLICATION

(11) **EP 4 043 047 A1**
(43) Date of publication of application: **17.08.2022**
(21) Application number: 21157244.1
(22) Date of filing: 15.02.2021
(51) Int. Cl.: A61M 5/142, A61B 5/145, A61B 5/00

(54) **INSERTION SYSTEM AND METHOD FOR INSERTING A MEDICAL DEVICE**

(71) Applicant: Roche Diabetes Care GmbH, 68305 Mannheim (DE); F. Hoffmann-La Roche AG, 4070 Basel (CH)
(72) Inventor: List, Hans, 68305 Mannheim (DE)
(74) Representative: Altmann Stößel Dick Patentanwälte PartG mbB

(57) **Abstract**

An insertion system (110) is disclosed. The insertion system (110) comprises a medical device (112) and an insertion device (113) for inserting the medical device (112) into a body tissue of a user, the insertion device (113) comprising:
i) an insertion component (118) configured for inserting the medical device (112) into the body tissue;
ii) an insertion component retractor (120);
iii) a cap (122);
iv) a guide sleeve (124) and an insertion sleeve (128) guided therein;
v) a locking sleeve (126) positioned in the insertion sleeve (128); and
vi) an elastic member (130) positioned between the locking sleeve (126) and the insertion component retractor (120);
wherein, for inserting the medical device (112), the cap (122), the insertion component retractor (120), the locking sleeve (126) and the insertion sleeve (128) are movable relative to the guide sleeve (124) from a distal position to a proximal position, wherein the insertion device (113) is separable from the medical device (112), wherein the insertion system (110) is configured such that a separation of the insertion device (113) from the medical device (112) releases a movement of the locking sleeve (126) from its proximal position to a further proximal position.

## Description

### Technical Field

The invention relates to an insertion system and a method for inserting a medical device into a body tissue of a user. The medical device may specifically be configured for detecting at least one analyte in a body fluid of the user. The insertion system and the method may be applied in the field of continuous monitoring of the analyte in the body fluid of the user, specifically in the field of home care and in the field of professional care, such as in hospitals. Other applications, however, are also feasible.

### Background art

Monitoring certain body functions, more particularly monitoring one or more analyte concentrations such as one or more metabolite concentrations in a body fluid of a user plays an important role in the prevention and treatment of various diseases. Such analytes can include by way of example, but not exclusively, glucose, lactate, cholesterol or other types of analytes and metabolites. Without restricting further possible applications, the invention will be described in the following text with reference to glucose monitoring. However, additionally or alternatively, the invention can also be applied to other types of analytes, such as the analytes mentioned above.

Generally, systems for long-term monitoring of an analyte in a body tissue of a user as well as corresponding insertion devices are known. For example, US 7,310,544 B2 discloses systems and methods for measuring an analyte in a host. More particularly, the invention relates to systems and methods for transcutaneous measurement of glucose in a host.

EP 1624914 discloses a device comprising a housing having a mounting surface adapted for application to the skin of a subject, a needle with a pointed end portion adapted to penetrate the skin of the subject, the needle having a first position in which the distal end portion is retracted within the housing, and a second position in which the distal end portion projects relative to the mounting surface. The device further comprises driving means actuatable to cause activation as well as release of the driving means, thereby moving the needle from the first position to the second position. By this arrangement the needle device can be supplied to the user in a non-energized state, the energizing taking place when the device is actuated by the user which means that energy will be stored only for a period from a few seconds to a few hours or days.

For monitoring an analyte in a body fluid, a sensor may be brought in contact with the body fluid. Generally, this requires inserting the sensor subcutaneously. There are systems, as an example, in which the sensor may be connected to an electronics unit upon insertion of the sensor. These systems may comprise a sensor base plate through which the sensor is inserted. After insertion of the sensor, the electronics unit may be connected to the base plate and the sensor. The base plate may reveal an exposed spot within the covered area and the sensor may be inserted into the skin of the user at the exposed spot. For systems in which the sensor is fixedly connected to the electronics unit, the insertion of the sensor may be performed simultaneously with the attachment of the electronics unit to the skin of the user.

Generally, the sensor may be inserted using an insertion component, such as an insertion needle. Often, such systems comprise a mechanism which retracts the insertion component after the electronics unit was attached to the skin of the user and the sensor was inserted into the skin of the user. The insertion component may be retracted deeply into the housing of the system to minimize the infection risk. The retraction of the insertion component may be initiated mechanically after insertion movement of the insertion component into the skin.

Despite the advantages achieved by the above-mentioned devices, several technical challenges remain. Commonly, it is difficult to insert the analyte sensor under the skin when it is firmly attached to the electronics unit with a larger footprint. Commonly, the system needs a relatively large opening through which the electronics unit may be pushed. In the opening a bulge in the skin may emerge. When the insertion needle hits the bulge, the skin may yield, a dent may emerge and the insertion needle cannot penetrate the skin.

### Problem to be solved

It is therefore desirable to provide an insertion system and a method for inserting a medical device into a body tissue of a user which at least partially address the above-mentioned technical challenges. Specifically, an insertion system is desirable which allows a safe and user-friendly handling of the insertion device while ensuring a reliable insertion of a medical device into a body tissue of a user.

### Summary

This problem is addressed by an insertion system and a method for inserting a medical device into a body tissue of a user with the features of the independent claims. Advantageous embodiments which might be realized in an isolated fashion or in any arbitrary combinations are listed in the dependent claims as well as throughout the specification.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect of the present invention, an insertion system is disclosed. The insertion system comprises a medical device and an insertion device for inserting the medical device into a body tissue of a user.

The term "system" as used herein is a broad term and is to be given its ordinary and customary meaning to a person of ordinary skill in the art and is not to be limited to a special or customized meaning. The term specifically may refer, without limitation, to a group of at least two elements which may interact with each other in order to fulfill at least one common function. The at least two components may be handled independently or may be coupled, connectable or integrable in order to form a common device. The term "insertion system" as used herein is a broad term and is to be given its ordinary and customary meaning to a person of ordinary skill in the art and is not to be limited to a special or customized meaning. The term specifically may refer, without limitation, to a group of at least two elements or components which are capable of interacting with each other in order to perform at least one transcutaneous or subcutaneous insertion of at least one of the two elements or components into a body tissue of a user, such as by performing an incision or a puncture in a skin of the user and by transferring the at least one of the two elements or components fully or partially into the body tissue.

The term "medical device" as used herein is a broad term and is to be given its ordinary and customary meaning to a person of ordinary skill in the art and is not to be limited to a special or customized meaning. The term specifically may refer, without limitation, to an arbitrary element or article being configured for use in the field of medical technology, specifically in the field of medical analytics or medical diagnostics. The medical device may be configured for performing at least one medical function and/or for being used in at least one medical process, such as one or more of a therapeutic process, a diagnostic process or another medical process.

The medical device may be configured to be mounted on a skin site of an extremity of the user. The extremity may be selected from the group consisting of: an arm, specifically an upper arm; a stomach; a shoulder; a back; hip; a leg. Specifically, the extremity may be the upper arm. However, also other applications may be feasible.

The medical device may comprise at least one component which may be configured to stay outside of the body tissue. Further, the medical device comprises at least one insertable portion. The insertable portion is configured for being inserted into the body tissue of the user. Specifically, the insertion device may be configured for inserting the insertable portion of the medical device into the body tissue of the user. Thus, "an insertion device for inserting the medical device into a body tissue of a user" in particular means "an insertion device for inserting the insertable portion of the medical device into the body tissue of the user".

As outlined above, the medical device may comprise the at least one insertable portion. The medical device, specifically the insertable portion of the medical device, may comprise at least one device selected from the group consisting of: an analyte sensor for detecting at least one analyte in a body fluid of the user, an infusion cannula and stimulating electrodes. Other embodiments may be feasible.

The analyte sensor for detecting at least one analyte in a body fluid of a user, such as in a body fluid contained in a body tissue of the user, may be configured for being used in qualitatively and/or quantitatively detecting the at least one analyte.

The term "analyte" as used herein is a broad term and is to be given its ordinary and customary meaning to a person of ordinary skill in the art and is not to be limited to a special or customized meaning. The term specifically may refer, without limitation, to a chemical and/or biological substance which takes part in the metabolism of the body of the user. Specifically, the analyte may be a metabolite or a combination of two or more metabolites. As an example, the analyte may be selected from the group consisting of: glucose, lactate, triglycerides, cholesterol. Still, other analytes or combinations of two or more analytes may be detected. The body tissue specifically may be or may comprise fatty tissue and/or interstitium. Other types of body tissue, however, are feasible.

The term "analyte sensor" as used herein is a broad term and is to be given its ordinary and customary meaning to a person of ordinary skill in the art and is not to be limited to a special or customized meaning. The term specifically may refer, without limitation, to a sensor which is capable of qualitatively or quantitatively detecting the presence and/or the concentration of the at least one analyte. The analyte sensor may be an electrochemical analyte sensor. The analyte sensor may comprise at least two electrodes. Specifically, the analyte sensor may comprise at least one two-electrode sensor. The two-electrode sensor may comprise precisely two electrodes, such as a working electrode and at least one further electrode such as a counter electrode, in particular a working electrode and a combined counter/reference electrode. The working electrode may comprise a working electrode pad and, optionally, at least one test chemical disposed thereon. The counter electrode may comprise a counter electrode pad. Additionally and optionally, one or more redox materials may be disposed thereon. The analyte sensor may further comprise one or more leads for electrically contacting the electrodes. The leads may, during insertion or at a later point in time, be connected to one or more electronic components. Preferably, the leads may already be connected to the electronic components before insertion of the analyte sensor. Further details on the electronic components are given below in more detail.

Specifically, the analyte sensor may be a needle-shaped or a strip-shaped analyte sensor having a flexible substrate and the electrodes disposed thereon. As an example, the analyte sensor may have a total length of 5 mm to 50 mm, specifically a total length of 7 mm to 30 mm. The term "total length" within the context of the present invention relates to the overall length of the analyte sensor which means a portion of the analyte sensor which is inserted and the portion of the analyte sensor which may stay outside of the body tissue. The portion of the analyte sensor which is inserted is also called the in-vivo portion, the portion of the analyte sensor which may stay outside of the body tissue is also called the ex vivo portion. Preferably, the in vivo portion has a length in the range from 3 mm to 12 mm. The analyte sensor may further comprise a biocompatible cover, such as a biocompatible membrane which fully or partially covers the analyte sensor and which prevents the test chemical from migrating into the body tissue and which allows for a diffusion of the body fluid and/or the analyte to the electrodes. Other embodiments of electrochemical analyte sensors, such as three-electrode sensors, may be feasible. For example, the three-electrode sensor may comprise, in addition to the working electrode and the counter electrode, a reference electrode.

In another embodiment, the analyte sensor may be an optical analyte sensor. For example, the analyte sensor may comprise a flexible light guide with glucose sensitive coating at its end and/or a tube like carrier with functional elements at inner or outer walls. Other embodiments of the analyte sensor may be possible too. For potential embodiments of analyte sensors, reference may be made to the above-mentioned prior art documents.

The term "infusion cannula" as used herein is a broad term and is to be given its ordinary and customary meaning to a person of ordinary skill in the art and is not to be limited to a special or customized meaning. The term specifically may refer, without limitation, to a hollow tube configured for delivering and/or infusing a medication into the body tissue of the user, in particular for delivering and/or infusing insulin into the body tissue of the user.

As outlined above, the medical device may comprise the at least one component which may be configured to stay outside of the body tissue. Specifically, the medical device may comprise at least one housing. The term "housing" as used herein is a broad term and is to be given its ordinary and customary meaning to a person of ordinary skill in the art and is not to be limited to a special or customized meaning. The term specifically may refer, without limitation, to an element or component having at least one interior space and at least one wall fully or partially surrounding the at least one interior space and providing protection to the interior space, such as one or more of a mechanical protection and a protection against environmental influences such as one or more of moisture, oxygen and microbial contaminations. The housing may generally be adapted to fully or partially surround and/or receive one or more elements in order to provide one or more of a mechanical protection, a mechanical stability, an environmental protection against moisture and/or ambient atmosphere, a shielding against electromagnetic influences or the like. The housing may also provide a basis for attachment and/or holding one or more further components or elements.

Specifically, the housing may be configured for holding one or more electronic components. Thus, the housing may also be referred to as an electronics unit. Specifically, in case the medical device is a medical device for detecting the analyte in the body fluid, the electronics unit may be configured for one or more of determining and/or controlling a detection of the analyte and/or transmitting measurement data to another component. Specifically, the electronics component may be configured for one or more of performing a measurement with the sensor, performing a voltage measurement, performing a current measurement, recording sensor signals, storing measurement signals and/or measurement data, transmitting sensor signals to another component. Thus, the electronics unit specifically may comprise at least one of: a voltmeter, an ammeter, a potentiostat, a voltage source, a current source, a signal receiver, a signal transmitter, an analog-digital converter, an electronic filter, a data storage device, an energy storage device. The housing may specifically be embodied as a closed electronics unit. The analyte sensor may be partially enclosed by the housing.

In an embodiment, the housing may comprise at least one patch. The patch specifically may comprise a plate which may be used as a support of other components of the medical device such as of the electronic components. Further, the patch may be configured for attaching the components of the medical device to the skin site of the user. For this purpose, the patch may comprise at least one adhesive surface and/or at least one adhesive strip or plaster.

However, in particular, the housing may not comprise a patch. In this case, the housing may further be configured for direct attachment to the skin site of the user. Thus, the housing may comprise at least one attachment component which is capable of forming a connection to the skin site, such as at least one adhesive surface and/or at least one adhesive strip or plaster.

The term "user" as used herein is a broad term and is to be given its ordinary and customary meaning to a person of ordinary skill in the art and is not to be limited to a special or customized meaning. The term specifically relates to a person intending to monitor an analyte value, such as a glucose value, in the person's body tissue and/or to deliver medication, such as insulin, into the person's body tissue. In an embodiment, the term specifically may refer, without limitation, to a person using the insertion device. However, in an embodiment, the person using the insertion device is different from the user. For example, the medical device may be inserted by a person different from the user into the user's body tissue. For example, the user may be a patient suffering from a disease, such as diabetes.

The term "inserting" as used herein is a broad term and is to be given its ordinary and customary meaning to a person of ordinary skill in the art and is not to be limited to a special or customized meaning. The term specifically may refer, without limitation, to an action or process of one or more of transcutaneously or subcutaneously implanting and/or positioning the medical device, specifically the insertable portion of the medical device, into the body tissue of the user. The medical device may fully or partially be inserted into the body tissue. The insertion of the medical device may be performed by using the insertion device. After insertion, the medical device or at least a part of the medical device may remain in the body tissue of the user for a predetermined period of time, such as for several hours, specifically for one or more days, more specifically for up to one week, even more specifically for up to two weeks or even more. The medical device may be configured for continuously monitoring and/or detecting the analyte in the body fluid of the user.

The term "insertion device" as used herein is a broad term and is to be given its ordinary and customary meaning to a person of ordinary skill in the art and is not to be limited to a special or customized meaning. The term specifically may refer, without limitation, to a device configured for inserting the medical device into the body tissue. The insertion device may be configured for transcutaneously or subcutaneously inserting the medical device into the body tissue, such as by performing an incision or a puncture in a skin of the user and by transferring the medical device fully or partially into the body tissue.

The insertion device comprises:
i) an insertion component configured for inserting the medical device into the body tissue;
ii) an insertion component retractor;
iii) a cap;
iv) a guide sleeve and an insertion sleeve guided therein;
v) a locking sleeve positioned in the insertion sleeve; and
vi) an elastic member positioned between the locking sleeve and the insertion component retractor.
For inserting the medical device, the cap, the insertion component retractor, the locking sleeve and the insertion sleeve are movable relative to the guide sleeve from a distal position to a proximal position. The insertion device is separable from the medical device. The insertion system is configured such that a separation of the insertion device from the medical device releases a movement of the locking sleeve from its proximal position to a further proximal position.

The term "insertion component" as used herein is a broad term and is to be given its ordinary and customary meaning to a person of ordinary skill in the art and is not to be limited to a special or customized meaning. The term specifically may refer, without limitation, to an arbitrary element which may be insertable at least partially into the body tissue, particularly in order to deliver or to transfer a further element. The insertion component may be configured for supporting the insertion of the medical device. Specifically, the insertion component may be configured for supporting the insertion of the insertable portion of the medical device. The insertion component may comprise a tip or a sharp end for inserting the medical device, specifically the insertable portion of the medical device, into the body tissue. The insertion component may be or may comprise an insertion cannula or an insertion needle. In the context of the present invention, the insertion component is considered as part of the insertion device. However, in particular during manufacturing of the insertion system, the insertion component may also be considered as a part of the medical device.

The term "insertion cannula" as used herein is a broad term and is to be given its ordinary and customary meaning to a person of ordinary skill in the art and is not to be limited to a special or customized meaning. The term specifically may refer, without limitation, to a hollow needle which may be at least partially slotted. The medical device may be received within the insertion cannula, such as within a lumen of the insertion cannula. The term "insertion needle" as used herein is a broad term and is to be given its ordinary and customary meaning to a person of ordinary skill in the art and is not to be limited to a special or customized meaning. The term specifically may refer, without limitation, to a compact needle, specifically without a slot and without any hollow parts. The medical device may be received on an outer surface of the insertion needle.

After insertion, the medical device, specifically the insertable portion of the medical device, may remain at least partially in the body tissue of the user. The insertion component, however, may be retracted from the body tissue of the user into the insertion device after inserting the medical device. For retracting the insertion component, the insertion device may comprise the insertion component retractor.

The term "insertion component retractor" as used herein is a broad term and is to be given its ordinary and customary meaning to a person of ordinary skill in the art and is not to be limited to a special or customized meaning. The term specifically may refer, without limitation, to at least one element of the insertion device configured for retracting the insertion component. The insertion component retractor may be configured for suspending the insertion component during an insertion movement and pull it out from the skin of the user during a retraction movement.

An engagement between the insertion component retractor and the insertion component may be loose, specifically not fastened. The engagement between the insertion component retractor and the insertion component may be established during a production process. The insertion component retractor may comprise at least one finger, gripper, hook, pincer or the like configured for retracting the insertion component. The finger, gripper, hook, pincer or the like may be arranged at a proximal end of the insertion component retractor, wherein, when the insertion device is in use, the proximal end of the insertion component retractor may point towards the body tissue of the user. For example, the insertion component retractor may comprise two or more fingers, grippers, hooks or pincers arranged symmetrically around the insertion component. For example, the insertion device may comprise at least one plunger or pull rod connected to the insertion component. The insertion component retractor may be connected to the plunger or push rod and/or may be configured for grabbing the plunger or pull rod in order to drive the insertion component to perform the retraction movement.

The insertion component retractor may further comprise the at least one wing. The term "wing" as used herein is a broad term and is to be given its ordinary and customary meaning to a person of ordinary skill in the art and is not to be limited to a special or customized meaning. The term specifically may refer, without limitation, to at least one element protruding outwards the insertion component retractor, in particular perpendicular to an insertion direction. A movement from the distal position to the proximal position refers to a movement in the insertion direction. The wing may protrude outwards from an outside of the insertion component retractor. For example, the insertion component retractor may form a cylindrical body. The wing may protrude from a lateral surface of the, in particular cylindrical, body of the insertion component retractor. In one embodiment, the insertion component retractor may comprise at least two wings. The two wings may be arranged opposite to each other at the outside of the insertion component retractor. The wing may comprise at least one inclined wing surface. The inclined wing surface may be tilted by an angle with respect to a direction of extension of the insertion component retractor. Further, the wing may have a shape of a prism, specifically of a triangular prism. The triangular prism may be based on a rectangular triangle. The interaction of the wing with other components of the insertion device will be outlined in further detail below.

The term "cap" as used herein is a broad term and is to be given its ordinary and customary meaning to a person of ordinary skill in the art and is not to be limited to a special or customized meaning. The term specifically may refer, without limitation, to an arbitrary shaped element configured for fully or partially enclosing one or more components of the insertion device and/or for providing protection for these one or more components, such as against mechanical influence and/or humidity. The cap may surround and/or may enclose fully or partially one or more further components, such as the insertion component retractor, the guide sleeve, the insertion sleeve, the locking sleeve, the elastic member, the insertion component and/or the medical device. The term "at least partially surround", also referred to as "at least partially enclose", as used herein is a broad term and is to be given its ordinary and customary meaning to a person of ordinary skill in the art and is not to be limited to a special or customized meaning. The term specifically may refer, without limitation, to embodiments wherein the cap fully surrounds the one or more further components of the insertion system, specifically of the insertion device, and to embodiments wherein the cap may surround at least a part of the one or more further components. For example, the cap of the insertion device may at least partially surround the insertion sleeve, the locking sleeve and the insertion component retractor. The cap may also at least partially surround the guide sleeve and, thus, in the proximal position, may fully cover the guide sleeve except for a proximal end of the guide sleeve. The cap may be arranged such that it surrounds and/or encloses the further components of the insertion system, wherein a proximal side of the insertion device may be at least partially uncovered by the cap allowing contacting the user's skin with the guide sleeve and movement of the insertion component out of the insertion device. The proximal side may be the side of the insertion device providing a contact area or region with the user's skin. A distal side may be a side of the insertion device opposite of the proximal side.

The cap may be or may comprise a rigid cap, such as a rigid cap made of one or more of a plastic material, a metallic material and a cardboard material. The cap specifically may be essentially rotationally symmetric, e.g. by having an axial rotational symmetry about an axis such as a cylinder axis or axis of extension. The term "essentially rotationally symmetric" as used herein is a broad term and is to be given its ordinary and customary meaning to a person of ordinary skill in the art and is not to be limited to a special or customized meaning. The term specifically may refer, without limitation, to the fact that the cap may be fully rotationally symmetric or may comprise at least one part being rotationally symmetric, whereas other parts of the cap may exhibit a form diverging from the rotational symmetry. The cap may be designed as a cylinder, a hemisphere or as a dome. The cap may have a shape that is adapted to the shape of the medical device. The cap may comprise an inner structure which may not be rotationally symmetric. An outer shape of the cap may also be asymmetrical, e.g. may be shaped ergonomically to be held by a user's hand. The inner structure of the cap may be cylindrical or prismatic corresponding to a structure of the insertion sleeve.

The cap may further comprise at least one cap latching element, specifically at the inner structure of the cap. The cap latching element may be configured for holding components of the insertion device together. Specifically, the cap latching element may interlock the cap with at least one of the other components, such as the guide sleeve, the insertion sleeve and/or the locking sleeve, in particular the insertion sleeve. The cap may be attached to the insertion sleeve during production. Specifically, the cap may be attached to the insertion sleeve by a snap-fit connection. However, additionally or alternatively, the cap may be attached to the insertion sleeve by gluing, welding, screwing, heat stacking or any other method known to one skilled in the art.

The term "guide sleeve" as used herein is a broad term and is to be given its ordinary and customary meaning to a person of ordinary skill in the art and is not to be limited to a special or customized meaning. The term specifically may refer, without limitation, to an enclosure of one or more components configured for guiding at least one movement of components enclosed by the guide sleeve, specifically of the insertion sleeve, the locking sleeve and the insertion component retractor. The guide sleeve may fully or partially enclose the insertion component retractor, the insertion component, the insertion sleeve, the locking sleeve and/or the elastic member. The guide sleeve may be partially enclosed by the cap.

The guide sleeve may be essentially rotationally symmetric, specifically in accordance with the symmetry of the cap of the insertion device, in particular of the inner structure of the cap of the insertion device. For example, in case the cap may have an axial rotational symmetry about an axis such as a cylinder axis or axis of extension, the guide sleeve may have a similar axial rotational symmetry. Specifically, the guide sleeve may have a shape which is adapted to a shape of the medical device.

The guide sleeve may be movable with respect to the cap of the insertion device. For example, when using the insertion device, the guide sleeve may be configured for sliding into the cap of the insertion device. The guide sleeve, in particular a proximal end of the guide sleeve, may be in contact with the user's skin when the insertion device is used.

The guide sleeve may comprise at least one inner guide sleeve latching element, specifically at an inner structure of the guide sleeve. The inner guide sleeve latching element of the guide sleeve may be arranged on an inner side of the guide sleeve. Specifically, the inner guide sleeve latching element may be arranged such that it faces the components enclosed by the guide sleeve, specifically the insertion sleeve. The inner guide sleeve latching element may be configured for holding components of the insertion device, specifically the insertion sleeve. The inner guide sleeve latching element may interlock the guide sleeve with the insertion sleeve, specifically when the insertion sleeve is in the proximal position.

The term "insertion sleeve" as used herein is a broad term and is to be given its ordinary and customary meaning to a person of ordinary skill in the art and is not to be limited to a special or customized meaning. The term specifically may refer, without limitation, to an element configured for at least partially enclosing the looking sleeve and/or the insertion component retractor. The insertion sleeve itself may be enclosed fully or partially by the guide sleeve and/or by the cap of the insertion device.

The insertion sleeve may be essentially rotationally symmetric, specifically in accordance with the symmetry of the cap and the guide sleeve. For example, the cap and the guide sleeve may have an axial rotational symmetry about an axis such as a cylinder axis or axis of extension, the insertion sleeve may have a similar axial rotational symmetry. The insertion sleeve may be configured for receiving the locking sleeve. For example, the insertion sleeve may comprise a rotational symmetric hollow center. The locking sleeve may be received at least partially within the rotational symmetric hollow center. The rotational symmetric hollow center may specifically be open to a distal end of the insertion sleeve and to a proximal end of the insertion sleeve. The proximal end may be opposite to the distal end of the insertion sleeve. The rotational symmetric hollow center may further be configured for receiving the medical device at least partially. The medical device may be arranged at the proximal end of the insertion sleeve.

Further, the insertion sleeve may comprise at least one insertion sleeve slot. The term "insertion sleeve slot" as used herein is a broad term and is to be given its ordinary and customary meaning to a person of ordinary skill in the art and is not to be limited to a special or customized meaning. The term specifically may refer, without limitation, to one or more of a groove, a trench cut and/or an opening of the insertion sleeve. The insertion sleeve slot may be a trench cut in the insertion sleeve such that the insertion sleeve slot may only partially cut into the insertion sleeve. The insertion sleeve slot of the insertion sleeve may extend essentially parallel to an axis of extension of the insertion sleeve. The term "essentially parallel" as used herein is a broad term and is to be given its ordinary and customary meaning to a person of ordinary skill in the art and is not to be limited to a special or customized meaning. The term specifically may refer, without limitation, to the fact that the insertion sleeve slot may extend parallel to the axis of extension of the insertion sleeve or, alternatively, may extend in a direction diverging from the axis of extension. The insertion sleeve slot may specifically be a straight slot. The term "straight" may refer to a continuous extension of the insertion sleeve slot in one direction without a bend, angle or curve. Consequently, the insertion sleeve slot may extend in one dimension. However, small aberrations of the insertion sleeve slot from the extension in one dimension may be existent specifically due to slight inaccuracies during manufacturing of the insertion sleeve.

Further, the insertion sleeve may comprise at least one inward protrusion. The term "inward protrusion" as used herein is a broad term and is to be given its ordinary and customary meaning to a person of ordinary skill in the art and is not to be limited to a special or customized meaning. The term specifically may refer, without limitation, to a protrusion of the insertion sleeve being protruding inwards the insertion sleeve. The inward protrusion may protrude from the insertion sleeve into an interior of the insertion sleeve. For example, the insertion sleeve may form a cylindrical body. The inward protrusion may protrude from a lateral surface of the, in particular cylindrical, body of the insertion sleeve. Specifically, the inward protrusion may be arranged such that it faces the components enclosed by the insertion sleeve, specifically the locking sleeve. The inward protrusion may be configured for interacting with components of the locking sleeve which will further be discussed below in more detail. Specifically, the insertion sleeve may comprise two inward protrusions which may be arranged opposite to each other in the interior of the insertion sleeve.

Further, the insertion sleeve may comprise one or more holding elements. As described above, the medical device may comprise the housing. The housing may be held by the holding elements. The term "holding element" as used herein is a broad term and is to be given its ordinary and customary meaning to a person of ordinary skill in the art and is not to be limited to a special or customized meaning. The term specifically may refer, without limitation, to an element of the insertion sleeve which is configured for engaging with the housing of the medical device, specifically of a surface of the medical device, for the purpose of supporting the housing and keep the housing in position. The holding element may be or may comprise at least one finger, gripper, hook, pincer or the like. Also other embodiments may be feasible. The holding element may be arranged at the inner structure of the insertion sleeve.

Further, the insertion sleeve may comprise at least one insertion sleeve latching element, The insertion sleeve latching element may be arranged on an outer side of the insertion sleeve. Specifically, the insertion sleeve latching element may be arranged such that it faces the guide sleeve. The insertion sleeve latching element may be configured for interacting with the inner guide sleeve latching element of the guide sleeve as described above, specifically when the insertion sleeve is in the proximal position.

The term "locking sleeve" as used herein is a broad term and is to be given its ordinary and customary meaning to a person of ordinary skill in the art and is not to be limited to a special or customized meaning. The term specifically may refer, without limitation, to at least one element of the insertion device configured for controlling a movement of the insertion component retractor. The locking sleeve may fully or partially enclose the insertion component retractor and/or the elastic member. The locking sleeve may be partially enclosed by the insertion sleeve and/or the cap.

The locking sleeve may be essentially rotationally symmetric, specifically in accordance with the symmetry of the insertion sleeve of the insertion device, in particular of the inner structure of the insertion sleeve of the insertion device. For example, in case the insertion sleeve may have an axial rotational symmetry about an axis such as a cylinder axis or axis of extension, the locking sleeve may have a similar axial rotational symmetry.

The locking sleeve may comprise at least one receptacle. The receptacle may be arranged at a distal end of the locking sleeve. The distal end of the locking sleeve may refer to a part of the insertion sleeve being distal to user's skin. The receptacle may have at least one opening. The insertion component and/or the insertion component retractor may extend at least partially through the opening. The term "at least partially extend" as used herein is a broad term and is to be given its ordinary and customary meaning to a person of ordinary skill in the art and is not to be limited to a special or customized meaning. The term specifically may refer, without limitation, to the fact that the insertion component retractor and/or the insertion component may fully extend through the opening of the receptacle or, alternatively, a part of the insertion component retractor and/or of the insertion component may extend through the opening of the receptacle. Specifically, the insertion component, in particular, the plunger fixedly connected to the insertion component, may at least partially extend through the opening such that at least one part of the insertion component may be arranged below the opening, wherein at least one other part may be arranged above the opening. "Below the opening" in this context means in a proximal position and "above the opening" in this context means in a distal position, wherein the proximal position and the distal position are defined relative to the user's skin when the insertion device is in use.

The locking sleeve may comprise at least one first locking sleeve slot. Further, the locking sleeve may comprise at least one second locking sleeve slot. Specifically, the locking sleeve may comprise a plurality of the first locking sleeve slots and a plurality of the second locking sleeve slots. The plurality of the first locking sleeve slots may be referred to as a first set of locking sleeve slots and the plurality of the second locking sleeve slots may be referred to as a second set of locking sleeve slots. Specifically, the locking sleeve may comprise at least two of the first locking sleeve slots and at least two of the second locking sleeve slots. The terms "first locking sleeve slot" and "second locking sleeve slot" may be considered as nomenclature only, without numbering or ranking the named elements, without specifying an order and without excluding a possibility that several kinds of first locking sleeve slots and second locking sleeve slots may be present. Further, additional locking sleeve slots such as one or more third locking sleeve slots may be present. Further, the terms "first locking sleeve slot" and "second locking sleeve slot" specifically may refer, without limitation, to one or more of grooves, trench cuts and/or openings of the locking sleeve. The first locking sleeve slot and/or the second locking sleeve slot may respectively be a trench cut in the locking sleeve such that the first locking sleeve slot and/or the second locking sleeve slot may only partially cut into the locking sleeve. Alternatively and/or additionally, the first locking sleeve slot and/or the second locking sleeve slot may be an opening of the locking sleeve. The first locking sleeve slot and/or the second locking insertion sleeve slot of the locking sleeve may extend essentially parallel to an axis of extension of the locking sleeve. The term "essentially parallel" as used herein is a broad term and is to be given its ordinary and customary meaning to a person of ordinary skill in the art and is not to be limited to a special or customized meaning. The term specifically may refer, without limitation, to the fact that the first locking sleeve slot and/or the second locking sleeve slot may extend parallel to the axis of extension of the locking sleeve or, alternatively, may extend in a direction diverging from the axis of extension. Specifically, the first locking sleeve slot and/or the second locking sleeve slot may extend parallel to the axis of extension and may vary in one or more locations from the direction parallel to the axis of extension. For example, the first locking sleeve slot and/or the second locking sleeve slot may comprise at least one edge and, thus, may diverge from the axis of extension at the location of the edge. The edge may be a z-shaped edge. Specifically, the first locking sleeve slot may be z-shaped. Additionally and/or alternatively, the first locking sleeve slot and/or the second locking sleeve slot may be tilted about an angle with respect to the axis of extension of the locking sleeve. Further, the first locking sleeve slot and/or the second locking sleeve slot may comprise at least one straight section. The term "straight section" may refer to a section of the first locking sleeve slot and/or the second locking sleeve slot which extends continuously essentially parallel to the axis of extension. The locking sleeve may comprise at least one distal end and at least one proximal end which may be opposite to the distal end of the locking sleeve. The first locking sleeve slot may be located at the distal end of the locking sleeve. Specifically, the first locking sleeve slot may be configured for controlling a movement of the retractor. The second locking sleeve slot may be located at the proximal end of the locking sleeve. Specifically, the second locking sleeve slot may be configured for steering a movement of the locking sleeve relatively to the insertion sleeve. Specifically, the second locking sleeve slot may be configured for interacting with the inward protrusion of the insertion sleeve.

The term "elastic member" as used herein is a broad term and is to be given its ordinary and customary meaning to a person of ordinary skill in the art and is not to be limited to a special or customized meaning. The term specifically may refer, without limitation, to an element which, when being compressed or stretched from its resting position, may exert a force opposing to the compression or stretch. The elastic member may be configured for applying the force to one or more further elements of the insertion device. Specifically, the elastic member may be compressed or stretched when the medical device is inserted into the body tissue or, alternatively, the elastic member may already be in a compressed or stretched state, such as in a pre-tensioned state, prior to use of the insertion device. The elastic member may be or may comprise at least one spring. Also other embodiments may be feasible.

As outlined above, for inserting the medical device, the cap, the insertion component retractor, the locking sleeve and the insertion sleeve are movable relative to the guide sleeve from the distal position to the proximal position. Further, also the insertion component and/or the medical device may be moveable relative to the guide sleeve from the distal position to the proximal position. The guide sleeve may be regarded as fix or non-moving component of the insertion device since the guide sleeve may be positioned on the user's skin. The other components, such as the medical device, the insertion component, the insertion component retractor, the insertion sleeve, the locking sleeve, the elastic member and the cap may move in a proximal direction relative to the skin of the user relative to the guide sleeve.

The term "distal position" as used herein is a broad term and is to be given its ordinary and customary meaning to a person of ordinary skill in the art and is not to be limited to a special or customized meaning. The term specifically may refer, without limitation, to a position specification indicating a position of the insertion device and/or any parts thereof in relation to the user in which the insertion component and/or the cap are furthermost from the proximal side of the insertion device. Specifically, for inserting the medical device, the insertion device may be brought into contact with a skin site of the user. The distal position may refer to a position being distanced to the skin site of the user. The distal position may be an initial position prior to the insertion movement of the insertion device and/or any parts thereof. Each component of the insertion device may have its own and/or individual distal position. For example, the cap, the insertion component retractor, the insertion sleeve and/or the locking sleeve may have their own and/or individual distal positions, respectively. Prior to insertion, the cap, the insertion component retractor, the insertion sleeve and the locking sleeve may be in their distal position and may be ready for inserting the medical device into the body tissue of the user. After insertion, the insertion component retractor may be moved back to its distal position and the insertion component may be retracted from the body tissue when the insertion component retractor may be back in its distal position.

The term "proximal position" as used herein is a broad term and is to be given its ordinary and customary meaning to a person of ordinary skill in the art and is not to be limited to a special or customized meaning. The term specifically may refer, without limitation, to a position specification indicating a position of the insertion device and/or any parts thereof in relation to the user in which the insertion component and/or the cap are closest to the proximal side of the insertion device. Specifically, for inserting the medical device, the insertion device may be brought into contact with the skin site of the user. The proximal position may refer to a position being in close proximity to the skin site of the user. Each component of the insertion device may have its own and/or individual proximal position. For example, the cap, the insertion component retractor, the locking sleeve and the insertion sleeve may have their own and/or individual proximal positions, respectively. In case the cap, the insertion component retractor, the locking sleeve and the insertion sleeve are in their proximal position, the medical device may be inserted into the body tissue of the user. During insertion of the medical device, the guide sleeve may be in contact with the skin site of the user and, thus, may be, during insertion, in its proximal position.

As outlined above, the insertion device is separable from the medical device. The term "separable" as used herein is a broad term and is to be given its ordinary and customary meaning to a person of ordinary skill in the art and is not to be limited to a special or customized meaning. The term specifically may refer, without limitation, to a property of the insertion device of being disengageable from the medical device. The disengagement of the insertion device from the medical device may be supported by the user. Exemplarily, the user may exert a force onto the insertion device such as by pulling on the insertion device, specifically on the cap. The medical device may specifically comprise the plaster and may be attached to the skin site of the user via adhesive forces. The separation of the insertion device from the medical device may be typically carried out after the insertion of the medical device. Specifically, the separation of the insertion device from the medical device may be typically carried out after the cap, the insertion component retractor, the insertion sleeve and/or the locking sleeve have been moved from the distal position to the proximal position. As outlined above, the insertion sleeve comprises the holding elements. The housing may be held by the holding elements. The separation of the insertion device from the medical device may include a releasing of the medical device, specifically of the housing of the medical device by the holding elements.

As outlined above, the insertion system is configured such that a separation of the insertion device from the medical device releases a movement of the locking sleeve from its proximal position to the further proximal position. The term "release of a movement" as used herein is a broad term and is to be given its ordinary and customary meaning to a person of ordinary skill in the art and is not to be limited to a special or customized meaning. The term specifically may refer, without limitation, to an allowance of a movement. Thus, before the insertion device is separated from the medical device, a movement of the locking sleeve from its proximal position to the further proximal position is not possible. Specifically, the elastic member may be configured such that a separation of the insertion device from the medical device initiates the movement of the locking sleeve from its proximal position to the further proximal position. The term "initiation of a movement" as used herein is a broad term and is to be given its ordinary and customary meaning to a person of ordinary skill in the art and is not to be limited to a special or customized meaning. The term specifically may refer, without limitation, to a triggering of a movement. Specifically, the elastic member may be configured for exerting a force to the locking sleeve in the proximal direction. Thus, before separation of the insertion device from the medical device, the cap, the insertion component retractor and the insertion sleeve may be at the proximal position. The separation of the insertion device from the medical device may cause a start of a movement of the locking sleeve from its proximal position to the further proximal position. The further proximal position of the locking sleeve is different from the proximal position. In the further proximal position a distance between the locking sleeve and a bottom edge of the guide sleeve is smaller than the distance between the locking sleeve and the bottom edge of the guide sleeve in the proximal position. Specifically, the separation of the insertion device from the medical device may cause a start of a movement of the locking sleeve relative to the insertion sleeve and/or the guide sleeve from the proximal position to the further proximal position.

The movement of the locking sleeve from its proximal position to the further proximal position may be a helical movement of the locking sleeve. The term "helical movement" as used herein is a broad term and is to be given its ordinary and customary meaning to a person of ordinary skill in the art and is not to be limited to a special or customized meaning. The term specifically may refer, without limitation, to a combination of a rotational movement and a linear movement of the locking sleeve. The helical movement may have a constant slope or may have a variable slope. The rotational movement may also be referred to as rotational component of the helical movement and the linear movement may also be referred to as linear component of the helical movement. The helical movement may also be referred to as screw like movement. The rotational movement may be a movement of the locking sleeve upon itself, e.g. a rotational movement about an axis of rotation being congruent with the axis of extension of the cylindrical body of the locking sleeve. Specifically, the locking sleeve may be essentially rotationally symmetric and the rotational movement may be movement around a symmetry axis of the locking sleeve. The rotational movement may be a twist about an angle less than 90°, specifically less than 45°, more specifically less than 15°. Other embodiments are feasible, wherein the rotational movement may be about an angle of more than 90° or even more than 180°. Due to the rotational movement of the locking sleeve, the insertion component retractor may be released. The linear movement may specifically be a linear movement along the axis of rotation as outlined above. Specifically, the helical movement of the locking sleeve may be a helical movement of the locking sleeve within the insertion sleeve. The helical movement may be guided by the second locking sleeve slots of the locking sleeve in interaction with the inward protrusion of the insertion sleeve.

The movement of the locking sleeve from its proximal position to the further proximal position, specifically the helical movement of the locking sleeve from its proximal position to the further proximal position, may release a movement of the insertion component retractor from its proximal position to a retracted position. The term "retracted position" as used herein is a broad term and is to be given its ordinary and customary meaning to a person of ordinary skill in the art and is not to be limited to a special or customized meaning. The term specifically may refer, without limitation, to a position of the insertion component retractor wherein the insertion component retractor is pulled back to the distal side of the insertion device. Thus, the movement of the locking sleeve from its proximal position to the further proximal position may release a movement of the insertion component retractor away from the skin site of the user into an interior of the insertion device. A direction of the movement of the insertion component retractor from its proximal position to the retracted position may be an opposite direction of the movement of the locking sleeve from its proximal position to the further proximal position. After the movement of the insertion component retractor from its proximal position to the retracted position, the insertion component may be fully received in the receptacle of the locking sleeve as descripted above.

As outlined above, the locking sleeve may comprise the at least one first locking sleeve slot. Further, as outlined above, the locking sleeve may comprise the at least one second locking sleeve slot. Further, as outlined above, the insertion component retractor may comprise the at least one wing. Further, as outlined above, the insertion sleeve may comprise the at least one insertion sleeve slot. For further details on the first locking sleeve slot, the second locking sleeve slot, the wing and the insertion sleeve slot reference may be made to the description above.

For inserting the medical device, the wing may be configured for protruding through the first locking sleeve slot of the locking sleeve and for engaging the insertion sleeve slot of the insertion sleeve, thereby blocking a movement, specifically a rotational movement, of the insertion component retractor. As outlined above, the insertion component retractor may be received in the receptacle of the locking sleeve. Further, as outlined above, the locking sleeve may be enclosed fully or partially by the insertion sleeve. Thus, the wing may be configured for being received by the first locking sleeve slot of the locking sleeve and by the insertion sleeve slot of the insertion sleeve at the same time. The term "blocking a movement" as used herein is a broad term and is to be given its ordinary and customary meaning to a person of ordinary skill in the art and is not to be limited to a special or customized meaning. The term specifically may refer, without limitation, to a property of the wing of preventing or inhibiting a movement of the insertion component retractor relative to other components of the insertion device, specifically relative to the cap, the guide sleeve and/or to the insertion sleeve. However, a movement of the insertion component retractor relative to the guide sleeve may still be possible. Specifically, the movement from the distal position to the proximal position may still be possible. Specifically, the wing may be configured for protruding through the first locking sleeve slot of the locking sleeve and for engaging the insertion sleeve slot of the insertion sleeve, thereby blocking a movement of the insertion component retractor before the insertion device is separated from the medical device. Thus, the wing may also be configured for protruding through the first locking sleeve slot of the locking sleeve and for engaging the insertion sleeve slot of the insertion sleeve, thereby blocking a movement of the insertion component retractor after insertion of the insertion component into the body tissue.

A movement of the locking sleeve relative to the insertion sleeve may be guided by an interaction of the inward protrusion of the insertion sleeve and the second locking sleeve slot of the locking sleeve. A shape of the second locking sleeve slot may be configured for enabling the helical movement as described above. As long as the linear component of the helical movement is blocked by the medical device the rotational component of the helical movement is also blocked.

As soon as the rotational component of the helical movement takes place, the first locking sleeve slot of the locking sleeve and the insertion sleeve slot get aligned such that the wing of the insertion component retractor is moveable. The elastic member may be configured for moving the insertion component retractor to its retracted position. Thereby the insertion component may be retracted into the insertion device.

As outlined above, the first locking sleeve slot may be z-shaped. Specifically, the first locking sleeve slot may have at least one inclined surface configured for blocking movement of the insertion component retractor. The inclined surface may be tilted by an angle with respect to a direction of extension of the locking sleeve. Further, as outlined above, the first locking sleeve slot may have at least one straight section. The wing of the insertion component retractor may rest with the inclined wing surface on the inclined surface of the first locking sleeve slot, thereby blocking the movement of the insertion component retractor. A rotational movement of the locking sleeve within the insertion sleeve may cause at least partial alignment of the first locking sleeve slot and the insertion sleeve slot such that the wing of the insertion component retractor is movable. The term "alignment" may refer to an arrangement in which the first locking sleeve slot and the insertion sleeve slot lie on top of each other. The term "at least partial alignment" may refer to an arrangement in which at least sections of the first locking sleeve slot and at least sections of the insertion sleeve slot lie on top of each other. Thus, the rotational movement of the locking sleeve within the insertion sleeve may cause the wing of the insertion component retractor being received in the straight section of the first locking sleeve slot and the wing may be moveable in the straight section of the first locking sleeve slot.

The elastic member may be positioned with its lower end on a bottom of the locking sleeve and with its upper end at a bottom of the insertion component retractor. In particular, the elastic member may be positioned within the receptacle of the locking sleeve. The terms "lower end" and "upper end" may refer to opposing ends of the elastic member. The lower end may refer to an end of the elastic member facing the skin site of the user. Specifically, the elastic member may be positioned with its lower end against the insertion locking sleeve and with its upper end against the insertion component retractor. The elastic member may specifically be the spring. The spring may be pre-tensioned, specifically before the insertion device is separated from the medical device. Further, the spring may be pre-tensioned before the medical device is inserted into the body tissue. The spring may specifically be trapped between the locking sleeve and the insertion component retractor as long as the medical device blocks the linear component of the helical movement of the locking sleeve, specifically relative to the insertion sleeve. The elastic member may be actuable by separating the insertion component from the medical device. Provided that the user applies the force to the cap, the elastic member may not be able to move the insertion component retractor from its proximal position to the retracted position. The elastic member may be configured for moving the insertion component retractor to its retracted position. Thereby the insertion component may be retracted into the insertion device.

As outlined above, the locking sleeve may comprise the at least one second locking sleeve slot. Further, as also outlined above, the insertion sleeve may comprise the at least one inward protrusion. The second locking sleeve slot may be configured for interacting with the inward protrusion. The rotational movement of the locking sleeve within the insertion sleeve may be blocked as long as the linear movement of the locking sleeve is blocked.

As outlined above, the insertion sleeve may comprise the holding elements. The housing may be held by the holding elements. The housing may be configured for blocking a movement of the locking sleeve in direction of the housing while the housing is held by the holding elements. Specifically, the movement of the locking sleeve in direction of the housing may be blocked if the medical device and the insertion device are connected. The housing may be configured for providing a pressure, specifically a counter-pressure on the locking sleeve. The insertion system may be configured for releasing the holding elements from the housing. The insertion device may be configured such that the separation of the insertion device from the medical device releases a movement of the locking sleeve in said direction. By separating the insertion device from the medical device, the pressure, specifically the counter pressure, on the locking sleeve may be lifted.

In a further aspect of the present invention, a method for inserting a medical device using the insertion system as described above or as will further be described below in more detail is disclosed. Thus, for possible definitions and options, reference may be made to the disclosure of the insertion system according to the present invention.

The method comprises the following steps which specifically may be performed in the given order. The method may comprise further method steps which are not listed.

The method comprises:
a) applying the insertion device to a user's skin,
b) applying a force to the cap of the insertion device so that the cap moves from a distal position to a proximal position,
c) removing the insertion device from the user's skin, thereby separating the insertion device from the medical device, wherein the separation releases a movement of the locking sleeve from its proximal position to a further proximal position.

In step a), the guide sleeve, in particular the proximal end of the guide sleeve, may be set in contact with the user's skin, specifically at an insertion site. Thus, the guide sleeve may be applied to the skin site of the user.

In step b), the force to the cap may be applied by action of the user. By moving the cap from the distal position to the proximal position, the insertion sleeve, the locking sleeve, the insertion component retractor, the insertion component, the medical device and the elastic member may be moved relative to the guide sleeve from the distal position to the proximal position. During step b), the insertion component may be inserted into the body tissue of the user, thereby inserting the medical device into the body tissue of the user. The force may be applied by the user of the insertion device before and during the insertion of the insertion component into the body tissue.

During step c), the insertion component may be withdrawn from the body tissue of the user. By separating the insertion device from the medical device, a pressure of the medical device on the locking sleeve may be lifted. The locking sleeve moves from the proximal position to the further proximal position and releases the movement of the insertion component retractor from its proximal position to the retracted position.

In a further aspect, an insertion system is disclosed. The insertion system comprises a medical device and an insertion device for inserting the medical device into a body tissue of a user. The insertion device comprises:
i) an insertion component configured for inserting the medical device into the body tissue;
ii) an insertion component retractor;
iii) a cap;
iv) a guide sleeve and an insertion sleeve guided therein;
v) a locking sleeve positioned in the insertion sleeve; and
vi) an elastic member positioned between the locking sleeve and the insertion component retractor;

For inserting the medical device, the cap, the insertion component retractor, the locking sleeve and the insertion sleeve are movable relative to the guide sleeve from a distal position to a proximal position. The insertion device is separable from the medical device. The insertion system is configured such that a separation of the insertion device from the medical device releases a movement of the insertion component retractor from its proximal position to a retracted position. For possible definitions and options of the insertion system, specifically of the insertion device and of the medical device, reference may be made to the disclosure of the insertion system, the insertion device and the medical device according to the present invention.

The methods and devices according to the present invention provide a large number of advantages over known methods and devices. Commonly, it is difficult to insert the analyte sensor under the skin when it is firmly attached to the electronics unit with a larger footprint. The insertion system needs a relatively large opening through which the electronics unit may be pushed. In the opening a bulge in the skin may emerge. When the insertion component hits the bulge, the skin may yield, a dent may emerge and the insertion component cannot penetrate the skin. To overcome this, pressure with a full contact area of the electronics unit to the skin may be applied before the insertion component is retracted.

Specifically, the insertion system may be based on a simple technique, be highly reliable as well as very specific. The user's skin may be tensioned more and for a longer period of time than with common insertion systems allowing the insertion component to penetrate the skin and to insert the medical device. This results in a more reliable insertion of the medical device. The components of the insertion device may be manufacturable in an easy manner.

Summarizing and without excluding further possible embodiments, the following embodiments may be envisaged:
Embodiment 1: An insertion system, the insertion system comprising a medical device and an insertion device for inserting the medical device into a body tissue of a user, the insertion device comprising:
   i) an insertion component configured for inserting the medical device into the body tissue;
   ii) an insertion component retractor;
   iii) a cap;
   iv) a guide sleeve and an insertion sleeve guided therein;
   v) a locking sleeve positioned in the insertion sleeve; and
   vi) an elastic member positioned between the locking sleeve and the insertion component retractor;
   wherein, for inserting the medical device, the cap, the insertion component retractor, the locking sleeve and the insertion sleeve are movable relative to the guide sleeve from a distal position to a proximal position, wherein the insertion device is separable from the medical device, wherein the insertion system is configured such that a separation of the insertion device from the medical device releases a movement of the locking sleeve from its proximal position to a further proximal position.
Embodiment 2: The insertion system according to embodiment 1, wherein the movement of the locking sleeve from its proximal position to the further proximal position is a helical movement of the locking sleeve.
Embodiment 3: The insertion system according to embodiment 1 or 2, wherein the movement of the locking sleeve from its proximal position to the further proximal position releases a movement of the insertion component retractor from its proximal position to a retracted position.
Embodiment 4: The insertion system according to any one of embodiments 1 to 3, wherein the locking sleeve comprises at least one first locking sleeve slot, wherein the insertion component retractor comprises at least one wing and wherein the insertion sleeve comprises at least one insertion sleeve slot.
Embodiment 5: The insertion system according to embodiment 4, wherein for inserting the medical device the wing is configured for protruding through the first locking sleeve slot and for engaging the insertion sleeve slot, thereby blocking a movement of the insertion component retractor.
Embodiment 6: The insertion system according to any one of embodiments 4 or 5, wherein the first locking sleeve slot is z-shaped.
Embodiment 7: The insertion system according to any one of embodiments 4 to 6, wherein the first locking sleeve slot has at least one inclined surface configured for blocking movement of the insertion component retractor.
Embodiment 8: The insertion system according to any one of embodiments 4 to 7, wherein a rotational movement of the locking sleeve within the insertion sleeve causes at least partial alignment of the first locking sleeve slot and the insertion sleeve slot such that the wing of the insertion component retractor is movable, wherein the elastic member is configured for moving the insertion component retractor to its retracted position, thereby the insertion component is retracted into the insertion device.
Embodiment 9: The insertion system according to any one of embodiments 1 to 7, wherein the locking sleeve comprises at least one second locking sleeve slot, wherein the insertion sleeve comprises at least one inward protrusion, wherein the second locking sleeve slot is configured for interacting with the inward protrusion, wherein a rotational movement of the locking sleeve within the insertion sleeve is blocked as long as a linear movement of the locking sleeve is blocked.
Embodiment 10: The insertion system according to any one of embodiments 1 to 9, wherein the elastic member comprises at least one spring.
Embodiment 11: The insertion system according to embodiment 10, wherein the spring is pre-tensioned.
Embodiment 12: The insertion system according to any one of embodiments 1 to 11, wherein the medical device comprises a housing and the insertion sleeve comprises holding elements, wherein the housing is held by the holding elements, wherein the housing is configured for blocking movement of the locking sleeve in direction of the housing while the housing is held by the holding elements.
Embodiment 13: The insertion system according to any one of embodiments 1 to 12, wherein the medical device comprises at least one device selected from the group consisting of an analyte sensor for detecting at least one analyte in a body fluid of a user, an infusion cannula.
Embodiment 14: A method for inserting a medical device using the insertion system according to any one of embodiments 1 to 13, the method comprising the steps:
   a) applying the insertion device to a user's skin,
   b) applying a force to the cap of the insertion device so that the cap moves from a distal position to a proximal position,
   c) removing the insertion device from the user's skin, thereby separating the insertion device from the medical device, wherein the separation releases a movement of the locking sleeve from its proximal position to a further proximal position.
Embodiment 15: An insertion system, the insertion system comprising a medical device, and an insertion device for inserting the medical device into a body tissue of a user, the insertion device comprising:
   i) an insertion component configured for inserting the medical device into the body tissue;
   ii) an insertion component retractor;
   iii) a cap;
   iv) a guide sleeve and an insertion sleeve guided therein;
   v) a locking sleeve positioned in the insertion sleeve; and
   vi) an elastic member positioned between the locking sleeve and the insertion component retractor;
   wherein, for inserting the medical device, the cap, the insertion component retractor, the locking sleeve and the insertion sleeve are movable relative to the guide sleeve from a distal position to a proximal position, wherein the insertion device is separable from the medical device, wherein the insertion system is configured such that a separation of the insertion device from the medical device releases a movement of the insertion component retractor from its proximal position to a retracted position.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figure 1: shows an exemplary embodiment of an insertion system according to the present invention in a longitudinal-sectional view;
- Figure 2: shows an exemplary embodiment of an insertion component retractor in a perspective view;
- Figure 3: shows an exemplary embodiment of a cap in a perspective view;
- Figure 4: shows an exemplary embodiment of a guide sleeve in a perspective view;
- Figure 5: shows an exemplary embodiment of an insertion sleeve in a perspective view;
- Figure 6: shows an exemplary embodiment of a locking sleeve in a perspective view; and
- Figures 7A to 7E: show an exemplary embodiment of an insertion system according to the present invention in different longitudinal-sectional views.

### Detailed description of the embodiments

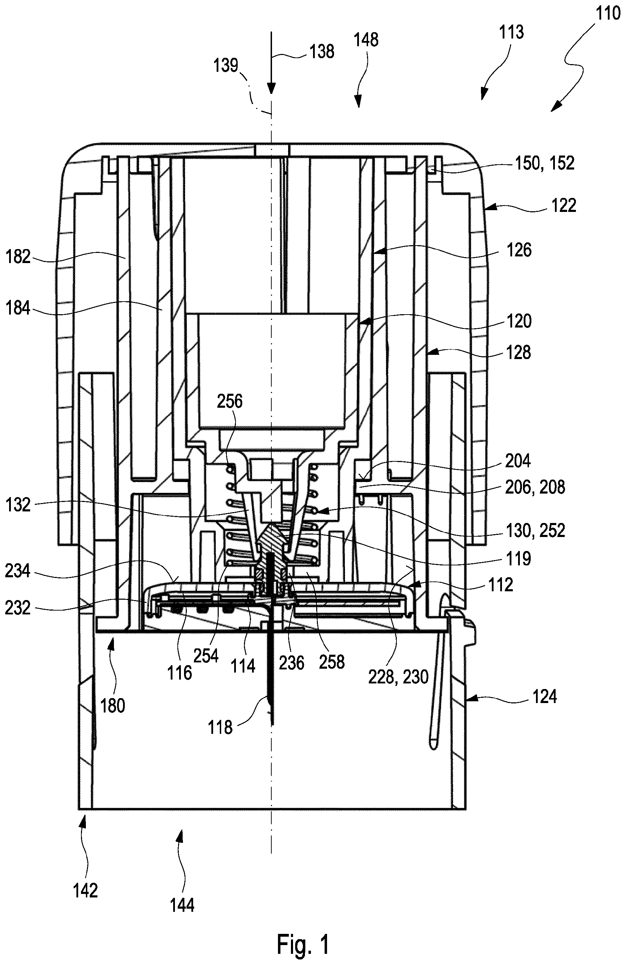

Figure 1 shows an exemplary embodiment of an insertion system 110. The insertion system 110 comprises a medical device 112 and an insertion device 113 for inserting the medical device 112 into a body tissue of a user according to the present invention. In Figure 1, the insertion system 110 is shown in a longitudinal-sectional view, wherein the longitudinal-sectional view passes through a median plane of the insertion system 110. Further, Figure 1 shows the insertion system 110 prior to insertion of the medical device 112.

The medical device 112 may be an arbitrary element or article being configured for use in the field of medical technology, specifically in the field of medical analytics or medical diagnostics. The medical device 112 may be configured for performing at least one medical function and/or for being used in at least one medical process, such as one or more of a therapeutic process, a diagnostic process or another medical process.

For example, the medical device 112 may be or may comprise at least one analyte sensor 114 for detecting at least one analyte in a body fluid of a user, such as in a body fluid contained in a body tissue of the user. The analyte sensor 114 may be configured for being used in qualitatively and/or quantitatively detecting the at least one analyte. The analyte may be a chemical and/or biological substance which takes part in the metabolism of the body of the user. Specifically, the analyte may be a metabolite or a combination of two or more metabolites. As an example, the analyte may be selected from the group consisting of: glucose, lactate, triglycerides, cholesterol. Still, other analytes or combinations of two or more analytes may be detected. The body tissue specifically may be or may comprise fatty tissue and/or interstitium. Other types of body tissue, however, are feasible.

The analyte sensor 114 may be a sensor which is capable of qualitatively or quantitatively detecting the presence and/or the concentration of the at least one analyte. For example, the analyte sensor 114 may be an electrochemical analyte sensor. The analyte sensor 114 may comprise at least two electrodes. Specifically, the analyte sensor 114 may comprise at least one two-electrode sensor. The two-electrode sensor may comprise precisely two electrodes, such as a working electrode and at least one further electrode such as a counter electrode, in particular a working electrode and a combined counter/reference electrode. The working electrode may comprise a working electrode pad and, optionally, at least one test chemical disposed thereon. The counter electrode may comprise a counter electrode pad. Additionally and optionally, one or more redox materials may be disposed thereon. The analyte sensor 114 may further comprise one or more leads for electrically contacting the electrodes. The leads may, during insertion or at a later point in time, be connected to an electronic component 116. Preferably, the leads are already connected to the electronics component 116 before insertion of the analyte sensor 114.

Specifically, the analyte sensor 114 may be a needle-shaped or a strip-shaped analyte sensor having a flexible substrate and the electrodes disposed thereon. As an example, the analyte sensor 114 may have a total length of 5 mm to 50 mm, specifically a total length of 7 mm to 30 mm. The analyte sensor 114 may further comprise a biocompatible cover, such as a biocompatible membrane which fully or partially covers the analyte sensor 114 and which prevents the test chemical from migrating into the body tissue and which allows for a diffusion of the body fluid and/or the analyte to the electrodes. Other embodiments of electrochemical analyte sensors 114, such as three-electrode sensors, may be feasible. For example, the three-electrode sensor may comprise, in addition to the working electrode and the counter electrode, a reference electrode.

In another embodiment, the analyte sensor 114 may be an optical analyte sensor. For example, the analyte sensor 114 may comprise a flexible light guide with glucose sensitive coating at its end and/or a tube like carrier with functional elements at inner or outer walls. Other embodiments of the analyte sensor 114 may be possible too. For potential embodiments of analyte sensors 114, reference may be made to the above-mentioned prior art documents.

In another embodiment not depicted here, for example, the medical device 112 may be or may comprise at least one infusion cannula. The infusion cannula may be or may comprise a hollow tube configured for delivering and/or infusing a medication, in particular insulin, into the body tissue of the user, in particular for delivering and/or infusing insulin into the body tissue of the user.

The user may be a person using the insertion system 110. The user may be a person intending to monitor an analyte value, such as a glucose value in the person's body tissue and/or to deliver medication, such as insulin into the person's body tissue. For example, the user may be a patient suffering from a disease, such as diabetes.

The inserting of the medical device 112 may comprise one or more of transcutaneously or subcutaneously implanting and/or positioning the medical device 112 into the body tissue of the user. The medical device 112, such as the analyte sensor 114, may fully or partially be inserted into the body tissue. The insertion of the medical device 112 may be performed by using the insertion device 113. The insertion device 113 may be configured for inserting the medical device 112 into the body tissue. The insertion device 113 may be configured for transcutaneously or subcutaneously inserting the medical device 112 into the body tissue, such as by performing an incision or a puncture in a skin of the user and by transferring the medical device 112 fully or partially into the body tissue. After insertion, the medical device 112 or at least a part of the medical device 112 may remain in the body tissue of the user for a predetermined period of time, such as for several hours, specifically for one or more days, more specifically for up to one week, even more specifically for up to two weeks or even more. The medical device 112 may be configured for continuously monitoring and/or detecting the analyte in the body fluid of the user.

The insertion device 113 comprises an insertion component 118, an insertion component retractor 120, a cap 122, a guide sleeve 124, a locking sleeve 126, an insertion sleeve 128 and an elastic member 130.

The insertion component 118 is configured for inserting the medical device 112 into the body tissue. The insertion component 118 may be insertable at least partially into the body tissue, particularly in order to deliver or to transfer a further element. The insertion component 118 may be configured for supporting the insertion of the medical device 112. The insertion component 118 may comprise a tip or a sharp end for inserting the medical device 112 into the body tissue. The insertion component 118 may be or may comprise an insertion cannula or an insertion needle.

As outlined above, after insertion, the medical device 112 may remain in the body tissue of the user. The insertion component 118, however, may be retracted from the body tissue of the user into the insertion device 113 after inserting the medical device 112. For retracting the insertion component 118, the insertion device 113 may comprise the insertion component retractor 120. An engagement between the insertion component retractor 120 and the insertion component 118 may be loose, specifically not fastened. The engagement between the insertion component retractor 120 and the insertion component 118 may be established during a production process.

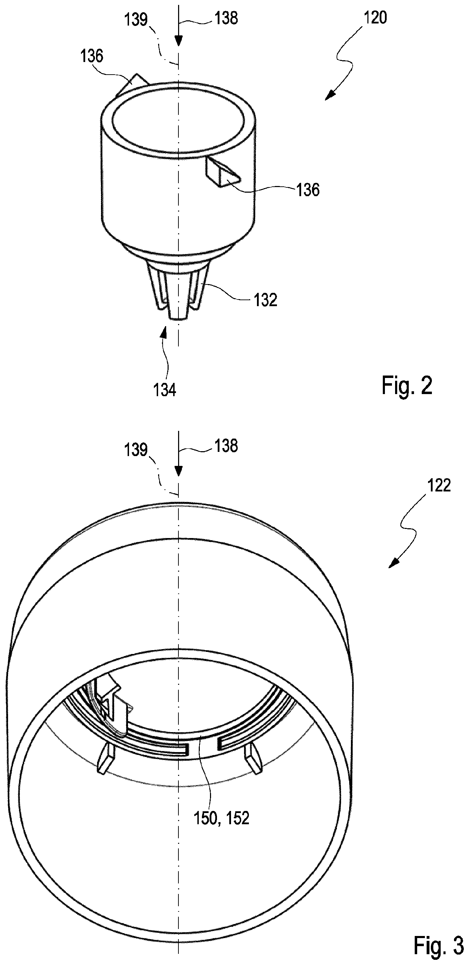

Figure 2 shows an exemplary embodiment of an insertion component retractor 120 in a perspective view.

The insertion component retractor 120 may comprise at least one finger, gripper, hook, pincer or the like configured for retracting the insertion component 118. For example, the insertion component retractor 120 may comprise two or more fingers, grippers, hooks or pincers arranged symmetrically around the insertion component 118 which is exemplarily depicted in Figure 1. The finger, gripper, hook, pincer or the like may be arranged at a proximal end 134 of the insertion component retractor 120, wherein, when the insertion system 110 is in use, the proximal end 134 of the insertion component retractor 120 may point towards the body tissue of the user (not shown). For example, in the exemplary embodiment shown in Figure 2, the insertion component retractor 120 may comprise three grippers 132. The grippers 132 may be arranged at the proximal end 134 of the insertion component retractor 120. The grippers 132 may be arranged symmetrically around the insertion component 118. For example, the insertion system 110 may comprise at least one plunger 119 connected to the insertion component 118. The plunger 119 is illustrated in Figure 1. The insertion component retractor 120 may be connected to the plunger 119 and/or may be configured for grabbing the plunger 119 to drive the insertion component 118 to perform the insertion movement and/or the retraction movement. Specifically, the grippers 132 may be configured for retracting the insertion component 118, specifically by being connected and/or by grabbing the plunger 119 as will be outlined in further detail below.

The insertion component retractor 120 may further comprise at least one wing 136. The wing 136 may be at least one element protruding outwards the insertion component retractor 120, in particular perpendicular to an insertion direction 138. The insertion component retractor 120 may form a cylindrical body. The cylindrical body may be disposed concentrically with respect to an axis of extension 139. The wing 136 may protrude outwards from a lateral surface of the cylindrical body. Specifically, the wing 136 may protrude perpendicular to the insertion direction 138, which may be essentially parallel with the axis of extension 139. In the embodiment of Figure 2, the insertion component retractor 120 may comprise at least two wings 136. The two wings 136 may be arranged opposite to each other at an outside of the insertion component retractor 120. The wing 136 may be configured for interacting with other components of the insertion device 113 which will be described in further detail below.

Figure 3 shows an exemplary embodiment of the cap 122 in a perspective view. The cap 122 may surround and/or may enclose fully or partially one or more further components, such as the insertion component retractor 120, the guide sleeve 124, the insertion sleeve 128, the locking sleeve 126, the elastic member 130 and/or the insertion component 118 such as illustrated in Figure 1. For example, as illustrated in Figure 1, the cap 122 may fully surround the insertion component retractor 120 and may partially surround the locking sleeve 126, the insertion sleeve 128 and the elastic member 130. The cap 122 may also at least partially surround the guide sleeve 124 and, thus, may fully cover the guide sleeve 124 except for a proximal end 142 of the guide sleeve 124. As shown in Figure 1, a proximal side 144 of the insertion device 113 may be at least partially uncovered by the cap 122 allowing contacting the user's skin with the guide sleeve 124 and movement of the insertion component 118 outside of the insertion device 113. The proximal side 144 may be the side of the insertion device 113 providing a contact area or region with the user's skin. A distal side 148 may be a side of the insertion device 113 opposite of the proximal side 144. The cap 122 may be or may comprise a rigid cap, such as a rigid cap made of one or more of a plastic material, a metallic material or a cardboard material.

The cap 122 specifically may be essentially rotationally symmetric, e.g. by having an axial rotational symmetry about an axis such as a cylinder axis or the axis of extension 139. The cap 122 may be designed as a cylinder, a hemisphere or as a dome. However, also other embodiments may be feasible. The cap 122 may have a shape which is adapted to a shape of the medical device 112. The cap 122 may comprise an inner structure 150 which may not be rotationally symmetric. An outer shape of the cap 122 may also be asymmetrical, e.g. may be shaped ergonomically to be held by a user's hand. The inner structure 150 of the cap 122 may be cylindrical or prismatic corresponding a structure of the insertion sleeve 128. The cap 122 may further comprise at least one latching element 152, specifically at the inner structure 150 of the cap 122. The latching element 152 may be configured for holding components of the insertion device 113 together. Specifically, the latching element 152 may interlock the cap 122 with at least one of the other components, such as the insertion sleeve 128.

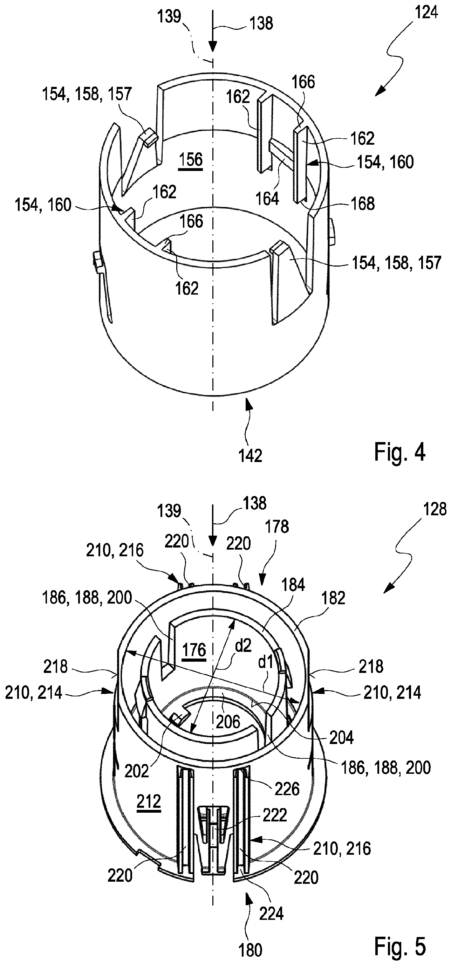

Figure 4 shows an exemplary embodiment of the guide sleeve 124 in a perspective view. The guide sleeve 124 may be essentially rotationally symmetric, specifically in accordance with the symmetry of the cap 122, in particular of the inner structure of the cap 122. For example, in case the cap 122 may have an axial rotational symmetry about an axis such as a cylinder axis or the axis of extension 139, the guide sleeve 124 may have a similar axial rotational symmetry. However, also other embodiments may be feasible. Specifically, the guide sleeve 124 may have a shape which is adapted to a shape of the medical device 112.

The guide sleeve 124 may be movable with respect to the cap 122. For example, when using the insertion device 113, the guide sleeve 124 may be configured for sliding into the cap 122. The guide sleeve 124, in particular the proximal end 142 of the guide sleeve 124, may be in contact with the user's skin when the insertion device 113 is used.

The guide sleeve 124 may comprise at least one inner guide sleeve latching element 154. The inner guide sleeve latching element 154 of the guide sleeve 124 may be arranged on an inner side 156 of the guide sleeve 124. Specifically, the inner guide sleeve latching element 154 may be arranged such that it faces the components enclosed by the guide sleeve 124, specifically the insertion sleeve 128. The inner guide sleeve latching element 154 may be configured for holding the insertion sleeve 128. The inner guide sleeve latching element 154 may interlock the guide sleeve 124 with the insertion sleeve 128. Specifically, the guide sleeve 124 may comprise at least two first inner guide sleeve latching elements 157, specifically at least two grippers 158. The grippers 158 may be arranged opposite to each other. Further, the grippers 158 may be inclined inwards. Further, the guide sleeve 124 may comprise at least two second inner guide sleeve latching elements 160. The second inner guide sleeve latching elements 160 may be arranged opposite to each other. The second inner guide sleeve latching elements 160 may respectively comprise one or more guide rails 162, specifically two guide rails 162. The guide rails 162 may extend essentially parallel to the axis of extension 139 of the guide sleeve 124. Further, the second inner guide sleeve latching elements 160 may respectively comprise at least one protrusion 164 which extends transverse, specifically perpendicular, to the guide rails 162. The protrusion 164 may be arranged between the two guide rails 162. Further, the protrusion 164 may be arranged in a distance to a first end 166 of the guide rails 162 and in a distance to a second end 168 of the guide rails 162. The interaction of the inner guide sleeve latching elements 154 with the insertion sleeve 128 may be described in further detail below.

As shown in Figure 1, the insertion sleeve 128 itself may be enclosed fully or partially by the guide sleeve 124 and the cap 122 of the insertion device 113.

Figure 5 shows an exemplary embodiment of the insertion sleeve 128 in a perspective view. The insertion sleeve 128 may be essentially rotationally symmetric, specifically in accordance with the symmetry of the cap 122 and the guide sleeve 124. For example, the cap 122 and the guide sleeve 124 may have an axial rotational symmetry about an axis such as a cylinder axis or the axis of extension 139, the insertion sleeve 128 may have a similar axial rotational symmetry.

The insertion sleeve 128 may be configured for receiving the locking sleeve 126, as illustrated in Figure 1. For example, the insertion sleeve 128 may comprise a rotational symmetric hollow center 176. The locking sleeve 126 may be received at least partially within the rotational symmetric hollow center 176. The rotational symmetric hollow center 176 may specifically be open to a distal end 178 of the insertion sleeve 128 and to a proximal end 180 of the insertion sleeve 128. The rotational symmetric hollow center 176 may further be configured for receiving the medical device 112 at least partially. The medical device 112 may be arranged at the proximal end 180 of the insertion sleeve 128, as illustrated in Figure 1.

Specifically, the insertion sleeve 128 may comprise at least one first cylindrically shaped component 182 and at least one second cylindrically shaped component 184. The first cylindrically shaped component 182 may have a first diameter *d₁* and the second cylindrically shaped component 184 may have a second diameter *d₂.* The first diameter *d₁* may be larger than the second diameter *d₂*. The second cylindrically shaped component 184 may be received at least partially in the first cylindrically shaped component 182. Specifically, the second cylindrically shaped component 184 and the first cylindrically shaped component 182 may be arranged essentially concentrically. The second cylindrically shaped component 184 may be configured for interacting with the locking sleeve 126. The first cylindrically shaped component 182 may be configured for attachment to the guide sleeve 124. The first cylindrically shaped component 182 may specifically be configured for stabilizing guide receptacles 220 which will further be described below in more detail.

Further, the insertion sleeve 128 may comprise at least one insertion sleeve slot 186. The insertion sleeve slot 186 may exemplarily be a trench cut 188 in the insertion sleeve 128 such that the insertion sleeve slot 186 may only partially cut into the insertion sleeve 128. Specifically, the insertion sleeve slot 186 may be part of the second cylindrically shaped component 184. Thus, exemplarily, the insertion sleeve slot 186 may be a trench cut 188 in the second cylindrically shaped component 184.

The insertion sleeve slot 186 of the insertion sleeve 128 may extend essentially parallel to the axis of extension 139 of the insertion sleeve 128. The insertion sleeve slot 186 may specifically be a straight slot 200. In the embodiment of Figure 5, the insertion sleeve 128 may comprise at least two insertion sleeve slots 186. The two insertion sleeve slots 186 may be arranged opposite to each other. The insertion sleeve slot 186 may be configured for interacting with the wings 136 of the insertion component retractor 120 which will be described in further detail below.

Further, the insertion sleeve 128 may comprise at least one inward protrusion 202. The inward protrusion 202 may protrude from the insertion sleeve 128 into an interior of the insertion sleeve 128. Specifically, the inward protrusion 202 may be arranged such that it faces the components enclosed by the insertion sleeve 128, specifically the locking sleeve 126. The inward protrusion 202 may be configured for interacting with components of the locking sleeve 126 which will further be discussed below in more detail. Specifically, the insertion sleeve 128 may comprise two inward protrusions 202 which may be arranged opposite to each other in the interior of the insertion sleeve 128. Specifically, the inward protrusion 202 may be part of the second cylindrically shaped component 184.

Further, the insertion sleeve 128 may comprise at least one support surface 204. Specifically, the insertion sleeve 128 may comprise a dead stop 206. The dead stop 206 may specifically be ring-shaped. The dead stop 206 may specifically be formed as a protrusion protruding from the insertion sleeve 128 into an interior of the insertion sleeve 128. The dead stop 206 may have the support surface 204. The dead stop 206 may specifically be configured for preventing the locking sleeve 126 from dropping out of the insertion sleeve 128. Further, the dead stop 206 may form a bottom of the second cylindrically shaped component 184 and may provide a cutout for a proximal end of the locking sleeve 126. When the locking sleeve 126 is in the distal position, the locking sleeve 126 may be arranged in a distance to the support surface 204. This is illustrated in Figure 1. When the locking sleeve 126 is in the proximal position, the locking sleeve 126 may be in direct contact with the support surface 204. The support surface 204 may be configured for blocking the locking sleeve 126. Specifically, the support surface 204 may be configured for preventing a further movement of the locking sleeve 126 in the insertion direction 138. Thus, in the further proximal position, the locking sleeve 126 may rest on the dead stop 206. The inward protrusion 202 may be arranged on the resting element 206. Specifically, the inward protrusion 202 and the dead stop 206 may be formed as one piece. Specifically, the dead stop 206 may be part of the second cylindrically shaped component 184. The dead stop 206 may be arranged at a lower end 208 of the second cylindrically shaped component 184 as illustrated in Figure 1.

Further, the insertion sleeve 128 may comprise at least one insertion sleeve latching element 210. The insertion sleeve latching element 210 may be arranged on an outer side 212 of the insertion sleeve 128. Specifically, the insertion sleeve latching element 210 may be arranged such that it faces the guide sleeve 124. The insertion sleeve latching element 210 may be configured for interacting with the inner guide sleeve latching element 154 of the guide sleeve as described above. Specifically, the insertion sleeve 128 may comprise at least one insertion sleeve latching element 214. The insertion sleeve latching element 214 may be configured for interacting with the first inner guide sleeve latching element 157, specifically with the gripper 158 which is as described above. Specifically, the insertion sleeve 128 may comprise at least two insertion sleeve latching elements 214. The at least two insertion sleeve latching elements 214 may be arranged opposite to each other. The insertion sleeve latching element 214 may specifically comprise at least one resting surface 218 on which the first inner guide sleeve latching element 157, specifically the gripper 158, may rest.

Further, the insertion sleeve 128 may comprise at least two insertion sleeve guide rails 216. The second insertion sleeve guide rails 216 may be configured for guiding a linear movement of the insertion sleeve 128 in the guide sleeve 124. The insertion sleeve guide rails 216 may be arranged opposite to each other. The insertion sleeve guide rails 216 may respectively comprise one or more guide receptacles 220. The guide receptacles 220 may extend essentially parallel to the axis of extension 139 of the insertion sleeve 128. Further, the insertion sleeve guide rails 216 may respectively comprise at least one receptacle 222 which extends transverse, specifically perpendicular, to the guide receptacles 220. The receptacle 222 may be arranged between the guide receptacles 220. Further, the receptacle 222 may be arranged in a distance to a first end 224 of the guide receptacles 220 and in a distance to a second end 226 of the guide receptacles 220.

When the insertion sleeve 128 moves relative to the guide sleeve 124 from the distal position to the proximal position, the insertion sleeve 128 may be slid at least partially into the guide sleeve 124. Thereby, the guide rails 162 of the second inner guide sleeve latching elements 160 of the guide sleeve 124 may slide in the guide receptacles 220 of the insertion sleeve guide rails 216. A sliding movement may be stopped when the protrusion 164 of the second inner guide sleeve latching elements 160 of the guide sleeve 124 is received in the receptacle 222 of the insertion sleeve guide rails 216 of the insertion sleeve 128. Further, in an assembled state of the guide sleeve 124 and the insertion sleeve 128 the grippers 158 may rest on the resting surface 218 of the insertion sleeve latching element 214 of the insertion sleeve 128.
Further, the insertion sleeve 128 may comprise one or more holding elements 228. As illustrated in Figure 1, an inner surface 230 of the insertion sleeve 128 may be formed as holding element 228. The inner surface 230 may be configured for engaging with a housing 232 of the medical device 112, specifically of a surface 234 of the housing 232 of the medical device 112.

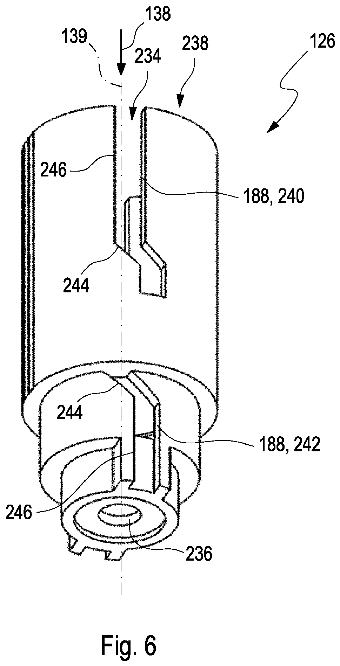

Figure 6 shows an exemplary embodiment of a locking sleeve 126 in a perspective view. The locking sleeve 126 may fully or partially enclose the insertion component retractor 120 as illustrated in Figure 1. The locking sleeve 126 may be partially enclosed by the insertion sleeve 128 and/or the cap 122 as illustrated in Figure 1. The locking sleeve 126 may be essentially rotationally symmetric, specifically in accordance with the symmetry of the insertion sleeve 128.

The locking sleeve 126 may comprise at least one receptacle 234. The receptacle 234 may be arranged at a distal end 238 of the locking sleeve. The receptacle 234 may have at least one opening 236. The insertion component 118 and/or the insertion component retractor 120 may extend at least partially through the opening 236.

The locking sleeve 126 may comprise the at least one first locking sleeve slot 240. Further, the locking sleeve 126 may comprise at least one second locking sleeve slot 242. The first locking sleeve slot 240 and/or the second locking sleeve slot 242 of the locking sleeve 126 may extend essentially parallel to the axis of extension 139 of the locking sleeve 126. The first locking sleeve slot 240 and/or the second locking sleeve slot 242 may comprise at least one edge 244 and, thus, may diverge from the axis of extension 139 at the location of the edge 244. The edge 244 may be a z-shaped edge. Further, the first locking sleeve 240 slot and/or the second locking sleeve slot 242 may comprise at least one straight section 246.

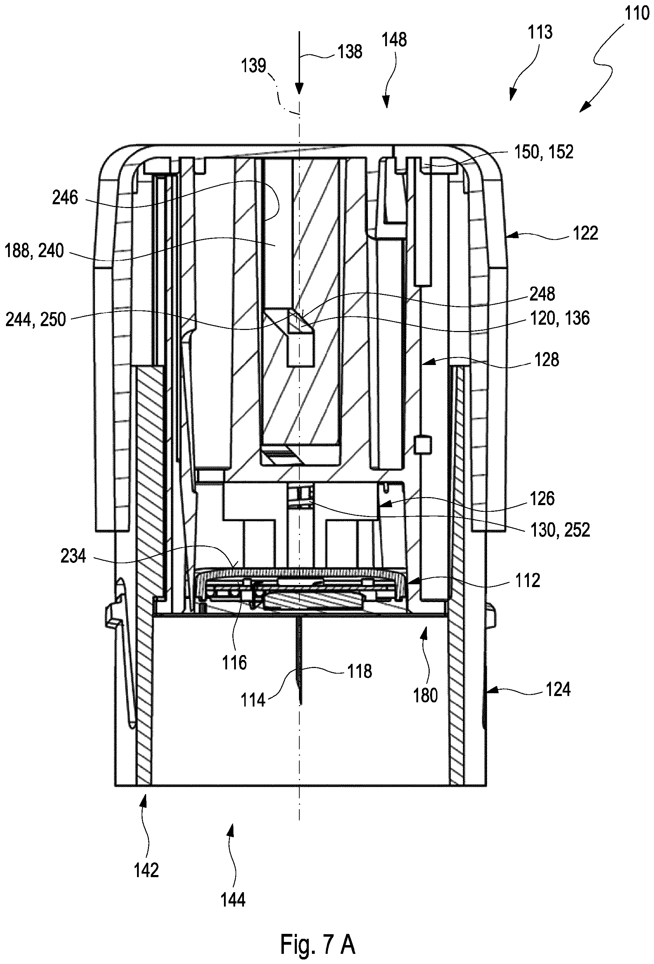

Figures 7A to 7E show an exemplary embodiment of an insertion system according to the present invention in different longitudinal-sectional views and during different stages of the insertion method. The embodiments according to Figures 7A to 7E correspond at least in large parts to the embodiment according to Figure 1. Further, the components of the insertion system 110 according to Figures 7A to 7E may correspond at least in large parts to the components as illustrated in Figures 2 to 6. Thus, reference to Figures 1 to 6 above is made.

As illustrated in Figure 7A, the locking sleeve 126 may be arranged in the insertion sleeve 128. Further, the insertion component retractor 120 may be arranged in the locking sleeve 126. The wings 136 of the insertion component retractor 120 may protrude through the first locking sleeve slot 240 of the locking sleeve 126. The first locking sleeve slot 240 may be z-shaped. Specifically, the first locking sleeve slot 240 may have the edge 244. The edge 244 may have an inclined surface 250. Specifically, the wing 136 may have an inclined wing surface 248. The inclined wing surface 248 of the wing 136 may rest on the inclined surface 250 of the edge 244 of the first locking sleeve slot 240.

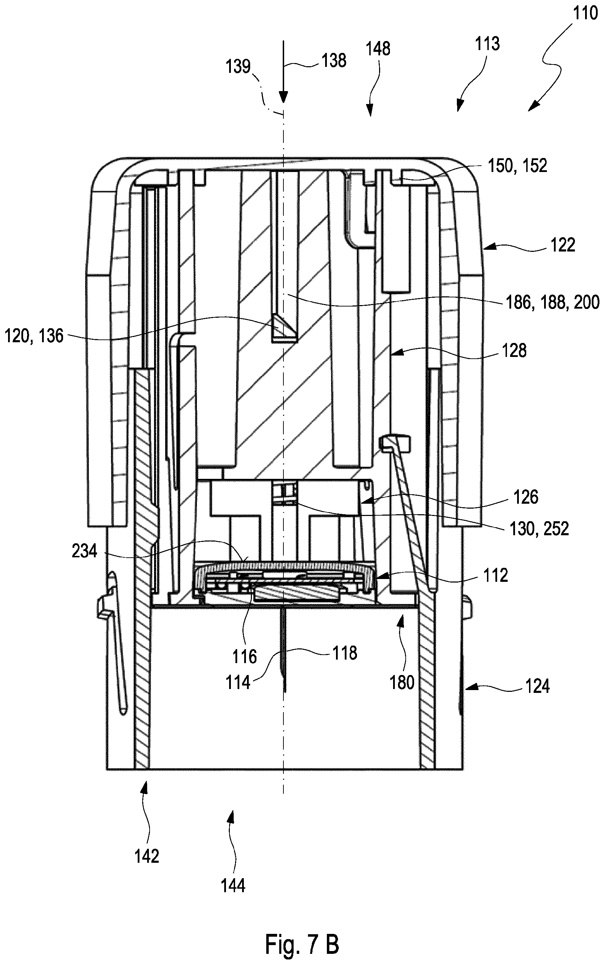

Further, as illustrated in Figure 7B, the wing 136 may engage with the insertion sleeve slot 186 of the insertion sleeve 128. Thus, a rotation of the insertion component retractor 120 may be blocked. Since the elastic member 130, which may specifically be embodied as a spring 252, rests with its lower end 254 on a bottom 258 of the locking sleeve 128 (see Figure 1) and stems its upper end 256 under the insertion element retractor 120, the insertion element retractor 120 and the locking sleeve 126 may be loaded and intend to rotate relative to each other. However, the wing 136 may be configured to block the movement of the insertion component retractor 120.

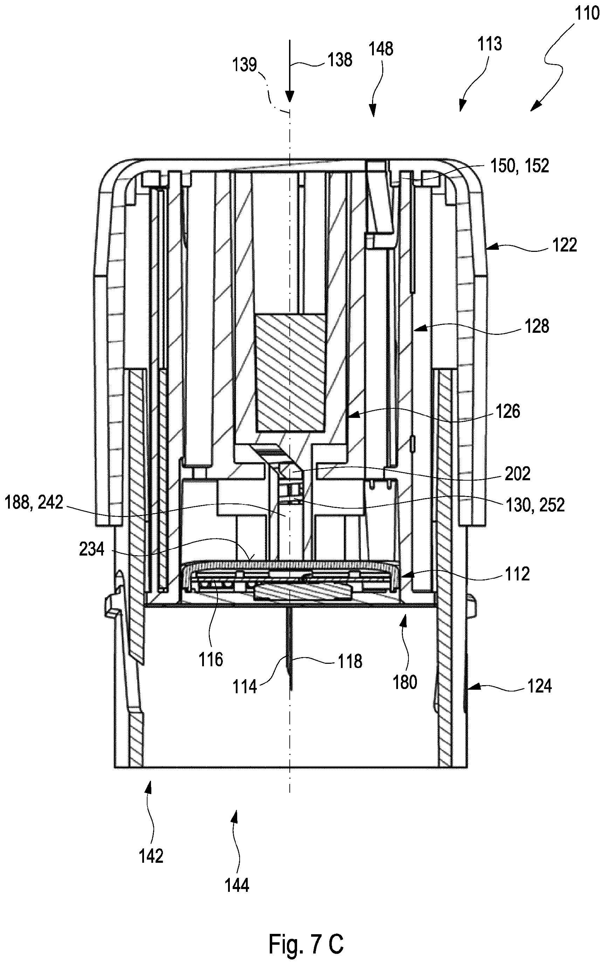

Further, as illustrated in Figure 7C, the locking sleeve 126 may comprise the at least one second locking sleeve slot 242. Further, as also outlined above, the insertion sleeve 128 may comprise the at least one inward protrusion 202. The second locking sleeve slot 242 may be configured for interacting with the inward protrusion 202. A rotational movement of the locking sleeve 126 within the insertion sleeve 128 may be blocked as long as a linear movement of the locking sleeve 126 is blocked.

The movement of the locking sleeve 126 may be blocked by the housing 232 of the medical device 112. The elastic member 130 may be pre-tensioned and the insertion system 110 as illustrated in Figure 7C may be in a cocked mode.

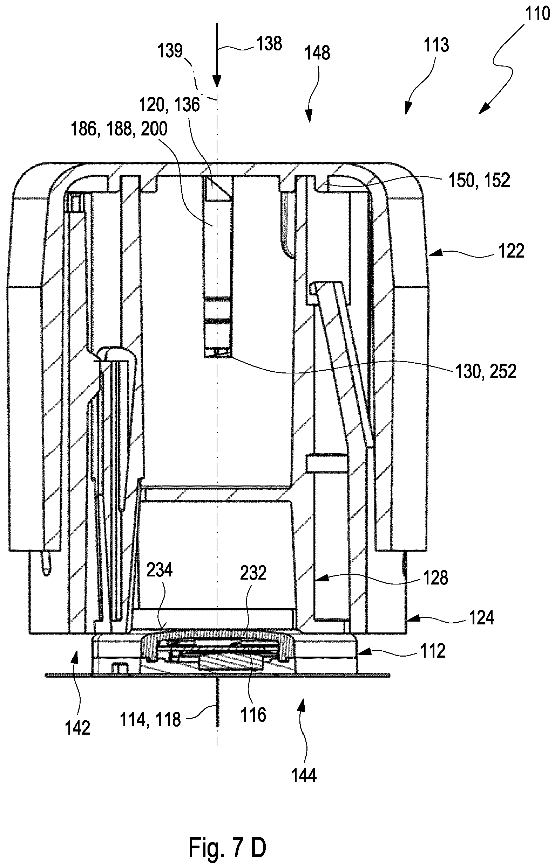

In Figure 7D, the insertion system 110 is shown in an inserted state. Thus, the medical device 112, the cap 122, the insertion component retractor 120, the locking sleeve 126 and the insertion sleeve 128 are moved relative to the guide sleeve 124 from the distal position, as illustrated in Figure 1 as well as in Figures 7A to 7C, to a proximal position as illustrated in Figure 7D. Even when the insertion sleeve 128 is released from the medical device 112, specifically from the housing 232 of the medical device 112, the elastic member 130 may be pre-tensioned. Further, the insertion system 110 may stay in the cocked mode. Thus, the wing 136 may be configured to block the movement of the insertion component retractor 120 within the locking sleeve 126.

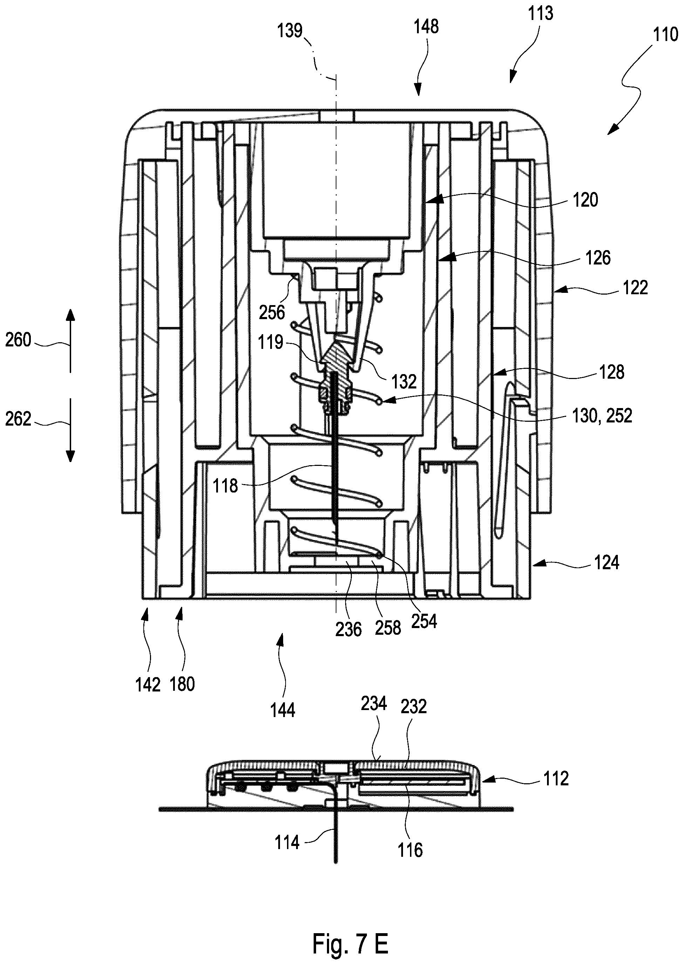

In Figure 7E, the insertion system 110 is separated from the medical device 112. A separation of the insertion device from the medical device releases a movement of the locking sleeve from its proximal position, as shown in Figure 7D, to a further proximal position. The further proximal position is illustrated in Figure 7E.

The movement of the locking sleeve 126 from its proximal position to the further proximal position may be a helical movement of the locking sleeve 126. Specifically, the helical movement of the locking sleeve 126 may be a helical movement of the locking sleeve 126 within the insertion sleeve 128. The movement of the locking sleeve 126 from its proximal position to the further proximal position may release a movement of the insertion component retractor 120 from its proximal position to a retracted position. Thus, the movement of the locking sleeve 126 from its proximal position to the further proximal position may release a movement of the insertion component retractor 120 away from the skin site of the user into an interior of the insertion device 113. A direction 260 of the movement of the insertion component retractor 120 from its proximal position to the retracted position may be an opposite direction 262 of the movement of the locking sleeve 126 from its proximal position to the further proximal position.

A rotational movement of the locking sleeve 126 within the insertion sleeve 128 may cause at least partial alignment of the first locking sleeve slot 240 and the insertion sleeve slot 186 such that the wing 136 of the insertion component retractor 120 is movable. Thus, the rotational movement of the locking sleeve 126 within the insertion sleeve 128 may cause the wing 136 of the insertion component retractor 120 being received in the straight section 246 of the first locking sleeve slot 240 and the wing 136 may be moveable in the straight section 246 of the first locking sleeve slot 240. The elastic member 130 may be actuable by separating the insertion component 118 from the medical device 112. The elastic member 130 may be configured for moving the insertion component retractor 120 to its retracted position. Thereby the insertion component 118 may be retracted into the insertion device 113.

After the movement of the insertion component retractor 120 from its proximal position to the retracted position, the insertion component 118 may be fully encircled by at least one of the locking sleeve 126, the insertion sleeve 128 and/or the guide sleeve 124. Further, the insertion component 118 may be fully received in the receptacle 234 of the locking sleeve 126 as descripted above.

### List of reference numbers

- 110: insertion system
- 112: medical device
- 113: insertion device
- 114: analyte sensor
- 116: electronic component
- 118: insertion component
- 119: plunger
- 120: insertion component retractor
- 122: cap
- 124: guide sleeve
- 126: locking sleeve
- 128: insertion sleeve
- 130: elastic member
- 132: gripper
- 134: proximal end
- 136: wing
- 138: insertion direction
- 139: axis of extension
- 142: proximal end
- 144: proximal side
- 148: distal side
- 150: inner structure
- 152: cap latching element
- 154: inner guide sleeve latching element
- 156: inner side
- 157: first inner guide sleeve latching element
- 158: gripper
- 160: second inner guide sleeve latching element
- 162: guide rail
- 164: protrusion
- 166: first end of the guide rail
- 168: second end of the guide rail
- 176: rotational symmetric hollow center
- 178: distal end of insertion sleeve
- 180: proximal end of insertion sleeve
- 182: first cylindrically shaped component
- 184: second cylindrically shaped component
- 186: insertion sleeve slot
- 188: trench cut
- 200: straight slot
- 202: inward protrusion
- 204: support surface
- 206: dead stop
- 208: lower end of the second cylindrically shaped component
- 210: insertion sleeve latching element
- 212: outer side
- 214: insertion sleeve latching element
- 216: insertion sleeve guide rail
- 218: resting surface
- 220: guide receptacle
- 222: receptacle of the insertion sleeve guide rail
- 224: first end of the guide receptacle
- 226: second end of the guide receptacle
- 228: holding element
- 230: inner surface
- 232: housing
- 234: receptacle of the locking sleeve
- 236: opening
- 238: distal end
- 240: first locking sleeve slot
- 242: second locking sleeve slot
- 244: edge
- 246: straight section
- 248: inclined wing surface
- 250: inclined surface
- 252: spring
- 254: lower end of the elastic member
- 256: upper end of the elastic member
- 258: bottom
- 260: direction of the movement of the insertion component retractor
- 262: direction of the movement of the locking sleeve

## Claims

1. An insertion system (110), the insertion system (110) comprising a medical device (112) and an insertion device (113) for inserting the medical device (112) into a body tissue of a user, the insertion device (113) comprising:
i) an insertion component (118) configured for inserting the medical device (112) into the body tissue;
ii) an insertion component retractor (120);
iii) a cap (122);
iv) a guide sleeve (124) and an insertion sleeve (128) guided therein;
v) a locking sleeve (126) positioned in the insertion sleeve (128); and
vi) an elastic member (130) positioned between the locking sleeve (126) and the insertion component retractor (120);
wherein, for inserting the medical device (112), the cap (122), the insertion component retractor (120), the locking sleeve (126) and the insertion sleeve (128) are movable relative to the guide sleeve (124) from a distal position to a proximal position, wherein the insertion device (113) is separable from the medical device (112), wherein the insertion system (110) is configured such that a separation of the insertion device (113) from the medical device (112) releases a movement of the locking sleeve (126) from its proximal position to a further proximal position.

2. The insertion system (110) according to claim 1, wherein the movement of the locking sleeve (126) from its proximal position to the further proximal position is a helical movement of the locking sleeve (126).

3. The insertion system (110) according to claim 1 or 2, wherein the movement of the locking sleeve (126) from its proximal position to the further proximal position releases a movement of the insertion component retractor (120) from its proximal position to a retracted position.

4. The insertion system (110) according to any one of claims 1 to 3, wherein the locking sleeve (126) comprises at least one first locking sleeve slot (240), wherein the insertion component retractor (120) comprises at least one wing (136) and wherein the insertion sleeve (128) comprises at least one insertion sleeve slot (186).

5. The insertion system (110) according to claim 4, wherein for inserting the medical device (112) the wing (136) is configured for protruding through the first locking sleeve slot (240) and for engaging the insertion sleeve slot (186), thereby blocking a movement of the insertion component retractor (120).

6. The insertion system (110) according to any one of claims 4 or 5, wherein the first locking sleeve slot (240) is z-shaped.

7. The insertion system (110) according to any one of claims 4 to 6, wherein the first locking sleeve slot (240) has at least one inclined surface configured for blocking movement of the insertion component retractor (120).

8. The insertion system (110) according to any one of claims 4 to 7, wherein a rotational movement of the locking sleeve (126) within the insertion sleeve (128) causes at least partial alignment of the first locking sleeve slot (240) and the insertion sleeve slot (186) such that the wing (136) of the insertion component retractor (120) is movable, wherein the elastic member (130) is configured for moving the insertion component retractor (120) to its retracted position, thereby the insertion component (118) is retracted into the insertion device (113).

9. The insertion system (110) according to claim 8, wherein the locking sleeve (126) comprises at least one second locking sleeve slot (242), wherein the insertion sleeve (128) comprises at least one inward protrusion (202), wherein the second locking sleeve slot (242) is configured for interacting with the inward protrusion (202), wherein a rotational movement of the locking sleeve (126) within the insertion sleeve (128) is blocked as long as a linear movement of the locking sleeve (126) is blocked.

10. The insertion system (110) according to any one of claims 1 to 9, wherein the elastic member (130) comprises at least one spring (252).

11. The insertion system (110) according to claim 10, wherein the spring (252) is pre-tensioned.

12. The insertion system (110) according to any one of claims 1 to 11, wherein the medical device (112) comprises a housing (232) and the insertion sleeve (128) comprises holding elements (228), wherein the housing (232) is held by the holding elements (228), wherein the housing (232) is configured for blocking movement of the locking sleeve (126) in direction of the housing (232) while the housing (232) is held by the holding elements (228).

13. The insertion system (110) according to any one of claims 1 to 12, wherein the medical device (112) comprises at least one device selected from the group consisting of an analyte sensor (114) for detecting at least one analyte in a body fluid of a user and an infusion cannula.

14. A method for inserting a medical device (112) using the insertion system (110) according to any one of claims 1 to 13, the method comprising the steps:
a) applying the insertion device (113) to a user's skin,
b)applying a force to the cap (122) of the insertion device (113) so that the cap (122) moves from a distal position to a proximal position,
c) removing the insertion device (113) from the user's skin, thereby separating the insertion device (113) from the medical device (112), wherein the separation releases a movement of the locking sleeve (126) from its proximal position to a further proximal position.
